# EUROPEAN PATENT SPECIFICATION

(11) **EP 1 304 966 B1**
(45) Date of publication and mention of the grant of the patent: **08.06.2005**
(21) Application number: 01961058.3
(22) Date of filing: 30.07.2001
(51) Int. Cl.: A61B 17/22

(54) **SYSTEM FOR ENHANCED CHEMICAL DEBRIDEMENT**
SYSTEM ZUM VERBESSERTEN CHEMISCHEN ENTFERNEN VON NEKROTISCHEM GEWEBE
SYSTEME DE DEBRIDEMENT CHIMIQUE AMELIORE

(30) Priority: 03.08.2000 IL 13768900
(43) Date of publication of application: 02.05.2003
(73) Proprietor: L.R.R & D Ltd., 94965 Omer (IL)
(72) Inventor: ROSENBERG, Lior, 84965 Omer (IL)
(74) Representative: Schmitz, Jean-Marie
(86) International application number: PCT/IL2001/000703
(87) International publication number: WO 2002/011624

(56) References cited:
- WO-A-98/32379
- WO-A-98/53850
- US-A- 4 982 730
- US-A- 5 618 275
- SCHAFER ET AL: "Experimental methods for ultrasonically enhanced wound healing" ULTRASONICS SYMPOSIUM, 1994. PROCEEDINGS., 1994 IEEE CANNES, FRANCE 1-4 NOV. 1994, NEW YORK, NY, USA,IEEE, US, 1 November 1994 (1994-11-01), pages 1853-1856, XP010139909 ISBN: 0-7803-2012-3
- KING W W K ET AL : "Debridement of burn wounds with a surgical ultrasonic aspirator" BURNS, vol. 22, no. 4, 1996, pages 307-309, XP001050524
- NICHTER L S ET AL: "Ultrasonic wound debridement" JOURNAL OF HAND SURGERY, vol. 13a, no. 1, 1988, pages 142-146, XP001050554

## Description

The present invention concerns a device for debridement of an area of skin to be debrided and the use of the device for the removal of dead tissue.

This invention relates to means for the removal of dead tissue, such as eschar and or necrotic tissue. More specifically, it relates to a device for the removal of such tissue, and the preparation of said device and its use for such purpose.

Various methods, features and compositions for the treatment of affected skin, particularly of diseased skin areas and burns, are known in the art.

US-A-4,372,296 claims a method of treating acne by topically administering ultrasonic vibrations to the acne-affected skin, together with the application of a composition of zinc sulfate and ascorbic acid and a pharmaceutical carrier which is an effective coupling agent for ultrasonic vibrations. This treatment may stimulate the production of collagen in the healing the scars.

US-A-5,656,015 describes an ultrasonic therapeutic system which comprises providing a plurality of spaced ultrasonic piezo-electric transducers, which may be so fixed to and around an afflicted area of a patient as to enable the sequential or interlaced excitation, whereby the affected area of the patient can be excited from different aspects. Each transducer delivers its ultrasonic energy via a body of material having a wave impedance that matches that of soft human tissue.

FR-A- 2 762 791 discloses a probe which permits the simultaneous application of ultrasound and electrical stimulations, for purposes of medical, paramedical or esthetic treatment.

WO 98/32379 describes an ultrasound system for the administration of a focused ultrasound beam to the body of a patient, comprising a container holding a liquid media, which container guides the ultrasound beam from the ultrasound generating element to the desired location of administration.

US-A-4,982,730 describes an ultrasound wound cleaning device having an annular transducer, an axial bore extending through said transducer through which liquid admixed with modifying agents can flow towards a surface to be treated.

S.F. Schoenbach et al., in an article in Plastic and Reconstructive Surgery, July 1980, 66(1), 34-37, describe tests carried out on rats for reducing the bacterial count of infected full-thickness burn wounds by ultrasonic treatment.

H.J. Klasen in "A review on the nonoperative removal of necrotic tissue from burn wounds", Burns 26 (2000), 207-222, offers a view of all non-surgical treatment of burns and concludes that enzymatic debridement would seem at first sight an attractive form of treatment, but unfortunately the results are highly variable. In particular, he describes developments regarding proteolytic enzymes including sutilains, collagenase, enzymes of plant origin and other agents.

Document WO-A-98/53850 discloses a device for debridement of an area of skin comprising retaining means adapted to be placed over the area of skin to be debrided, said retaining means containing as debriding agent, an opening and a seal.

The diseased or necrotic tissue or eschar, which has formed on the skin of a person for any reason, will be briefly designated hereafter as "dead tissue", and all such kinds of tissue should be understood as being comprised in said designation. Its removal is carried out, in the present state of the art, by mechanical means in an operation called debridement, which may be aided by the use of debriding agents. However, the art has not disclosed any apparatus or means that are effective for the removal of dead tissue and that do not require some degree of surgical debridement.

Accordingly, it is a purpose of this invention to provide a device that is effective in removing dead tissue, particularly necrotic and/or eschar tissue, that has formed on/in the tissue of a person, without surgical debridement.

Another aspect of the invention concerns the use of a novel device for the removal of dead tissue, particularly eschar tissue.

Other purposes and advantages of the invention will appear as the description proceeds.

The device of the present invention is described in the claim 1 and the use of said device is described in claim 16.

The invention provides a device for the removal of dead tissue from a person, which comprises liquid-retaining means, a liquid debriding agent retained by said retaining means, and an ultrasound probe coupled to said retaining means. Said device generates a novel combination of ultrasonic waves and chemical debriding agents.

The retaining means can be an acoustic chamber or a gel block or any other structural means, that is adapted to retain the liquid debriding agent peripherally, at least after it has been applied to the area that needs debridement. The retaining means must allow the debriding agent freely to contact the dead tissue, and therefore is open at the bottom, and may, in some embodiments, be open at the top. By "bottom" of the device is meant the part that contacts the dead tissue when the device is used, viz. the device will be described as if the treated portion of the patient tissue were substantially or approximately horizontal.

The liquid debriding agent may be chosen from efficacious debriding agents. Examples of such agents presently known to the medical art will be listed hereinafter.

Bromelain derivatives (i.e. Debridase, Ananaim, Comosain), Papain derivatives, bacterial derivative enzymes such as streptokinase (Varidase©), Sutilains (Travase©), Collagenase, Trypsin, Fibrolysin-desoxyribonuclease or chemical combinations such as "Aserbin cream©", acids such as Piruric or phosphoric acids.

More particularly, the device of the invention has the following components:
a) a piezo-electric/ultrasound source unit (hereinafter, briefly, USS) connected to a power source;
b) spacing means for keeping the USS at the right distance from the tissue to be treated;
c) an acoustic chamber containing the debriding agent and an acoustic medium;
d) sealing means between the lower edges of the acoustic chamber and the patient's tissues ("adhesive barrier");
e) positioning means for holding the device over the dead tissue and preventing its undesirable displacement;
f) a chemical or enzymatic debriding agent retained in the acoustic chamber; and
g) a solvent for the debriding agent, acting as an acoustic medium to promote the propagation of the ultrasound waves, in the form of fluid or gel also retained in the acoustic chamber.

The spacing means may consist in external support for the USS to keep it at the right distance from the tissue or may be constituted by the acoustic chamber itself.

The acoustic chamber may be rigid or soft, e.g., in the form of a flexible plastic body. It may have an inlet port for the debriding agent and/or the solvent and/or an outlet port to produce vacuum to secure the acoustic chamber to the tissue, when so desired, and/or to remove fluids from the chamber. Since the solvent also acts as an acoustic medium, hereinafter it will be called solvent-acoustic medium, or, briefly, solvent-medium.

The sealing means must have sufficient adhesion to prevent undesired displacement of the acoustic chamber during the treatment. In an embodiment of the invention they may be constituted by a seal body, such as a polyurethane foam element or an inflatable cushion or the like, which may be provided with a biocompatible adhesive such as an acrylic glue or a thick gel-like substance, to increase the adherence of the seal body to the skin and seal the acoustic chamber.

The positioning means may be the same as the sealing means or may be different. In an embodiment of the invention, they are constituted by several suction ventoses positioned around the acoustic chamber or a single large ventose that covers entirely the acoustic chamber or constituting a round elastic funnel around the chamber edges. Alternatively, the entire acoustic chamber may constitute a suction cup. As a further alternative, mechanical means such as elastic bandages, straps or adhesive tapes or the like can be used to position the acoustic chamber. When the positioning means are the same as the sealing means an adhesive layer under the sealing means may hold the acoustic chamber into place.

The solvent medium may be provided as part of the device before this latter is used, or may be introduced through an inlet port into the acoustic chamber after this latter is positioned and sealed over to the area to be treated.

The ultrasound probe must deliver ultrasound having a frequency measured in Hz and power measured in J/sm² (W/cm²), for example 20 kHz, and should deliver the ultrasound with a power, for example 10 J/sm² (W/cm²) Another aspect of the invention is the use of a device comprising a liquid debriding agent retained in retaining means and an ultrasound probe connected to a power source, for theremoval of dead tissue from a patien's body. The retaining means, as has been said, retain the liquid at the periphery, being open at the bottom and possibly at the top as well.

It should also be clear that the device of the invention can be used for purposes other than the removal of dead tissue.

In the drawings:
Figs. 1 to 4 are schematic vertical cross-sections illustrating various embodiments of the invention, "vertical cross-section" meaning herein a cross-section on a plane which is substantially perpendicular to the surface of the area being treated, and is a symmetry plane of the device.
Figs. 5 to 9 are photographs, illustrating an embodiment of the invention, on which a burn area covered by a dry eschar is shown in various stages of treatment.

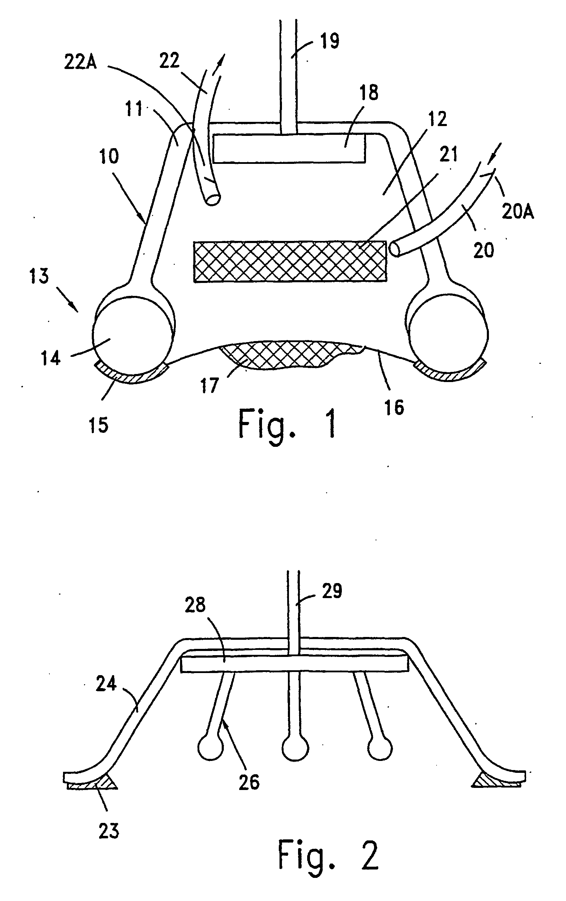

In Fig. 1, 10 generally indicates a device according to an embodiment of the invention. Since the device is round in shape, all vertical cross-sections are the same and Figs.1 to 4 represent any such cross-section. The device of Fig. 1 comprises a round cap 11 which is made of any convenient material, e.g., a plastic, which cup defines an acoustic chamber indicated at 12. At the bottom of cup 11 and around its periphery is located a seal generally indicated at 13. This seal comprises a body 14, preferably made of elastic foam, and an adhesive layer 15 which contacts the skin of the patient when the device is in use and retains it in the appropriate position With sufficient adhesive strength. For purposes of illustration, the skin of the patient is schematically indicated at 16 and the dead tissue is schematically indicated at 17. Obviously, the device is so placed as to overlay the dead tissue 17. The schematic indication of the skin and dead tissue is not repeated in the following drawings, but should be considered as obviously implicit, in view of the use of the device, which is the same in all illustrated embodiments.

Cup 11 is closed at the top and supports at the top of acoustic chamber 12 portion a USS 18, connected by conductors 19, passing through an opening (sealed if desired) at the top of cup 11, to a power source not shown. In this embodiment an inlet conduit 20 is shown for the introduction of debriding agent and solvent-acoustic medium into the acoustic chamber. The conduit may have a one-way valve 20A. However, the debriding agent can be provided as a unit 21 inserted into the acoustic chamber. 22 is an outlet for creating a vacuum, if desired, in the acoustic chamber to holding against the patient's body and may contain a one-way valve 22A. Since the USS 18 is connected to the inside of the top of container 11, so that its distance from the scar tissue 17 is determined by the height of the acoustic chamber.

Fig. 2 schematically illustrates a device in which a cup 24 that can be made of any flexible film is provided with a peripheral adhesive barrier 23. USS 28 (connected to a power source, not illustrated, by conductors 29) is supported by a spacer 26, illustrated in Fig. 2 as a tripod, but having any other convenient structure. The spacer determines the distance of the USS from the dead tissue to be treated. This embodiment can be held in place by elastic dressing, or tapes applied on top of it (not shown).

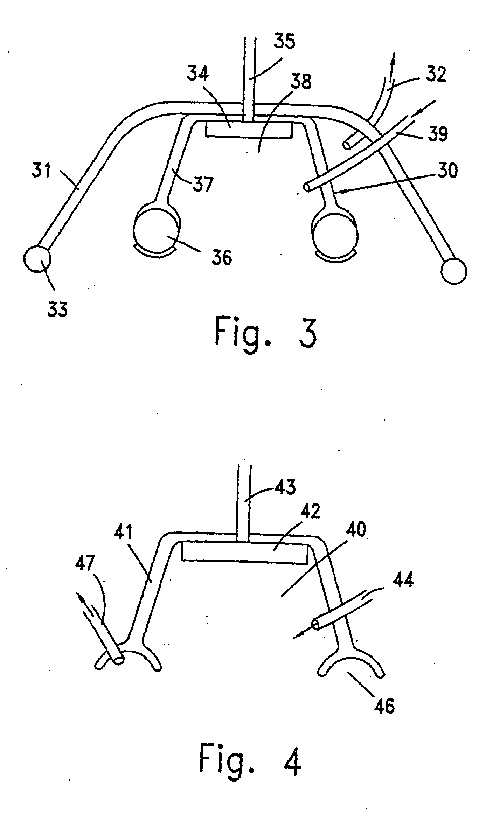

Fig. 3 illustrates an embodiment in which a device, generally indicated at 30 and similar in structure to the device 10 of Fig. 1 is held in place by an overlaying element 31, which may be called an anchoring ventose, and which is positioned on the area to be treated by applying a vacuum through an outlet 32. Anchor 31 is preferably provided with sealing means 33, to permit a vacuum being established therein. The device 30 comprises, like the device 10 of Fig. 1, a cup 37, which defines an acoustic chamber 38, supports a USS 34 provided with conductors 35, and is provided with a peripheral seal 36 similar to seal 13 of Fig. 1 which isolates the acoustic chamber 38 from the compartment between overlying element 31 and cup 37, in which a vacuum is created.

Fig. 4 shows an acoustic chamber 40 defined by a cup 41 and retaining a USS 42 with conductors 43. Inlet 44 permits to introduce a debriding agent and the solvent-medium into chamber 40. The sealing and positioning means in this embodiment are provided by annular cavity 46 at the periphery of the bottom of cup 41, in which a vacuum can be created through outlet 47, so that the said annular cavity acts as a ventose to secure the device into place.

The device of the invention may contain, as it is, all its components. The USS may be mounted in it, and the debriding agent and the solvent-acoustic medium be held therein in the form of a consistent component, e.g. retained in a porous or gel-like support. However, it may generated at the moment of use by conveniently combining its various components. Thus, the USS may be supported by an outside support, that is not part of the device, but which permits placing it at the appropriate distance from the dead tissue to be removed; and/or the debriding agent and the solvent-acoustic medium may be introduced into the acoustic chamber through an inlet at the moment of use.

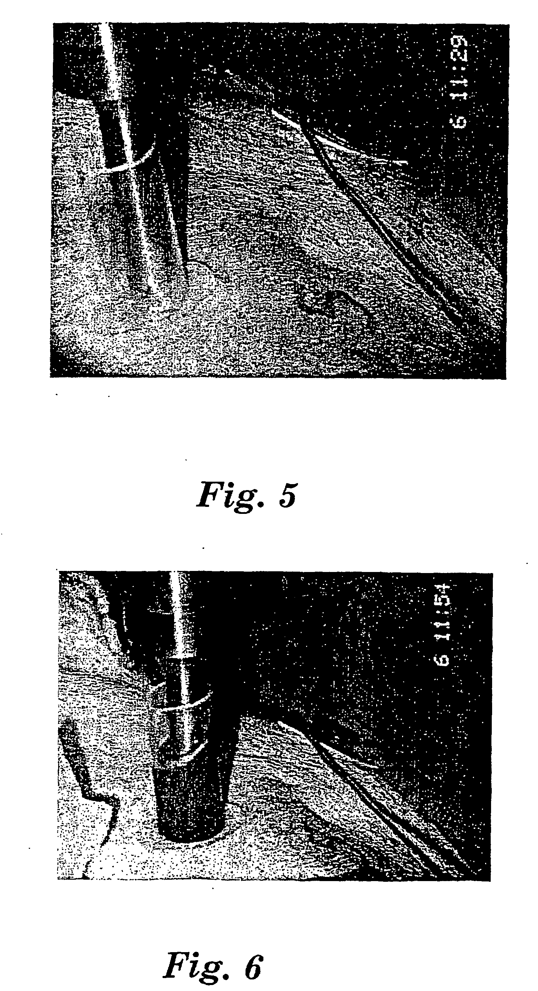

Figs. 5 to 9 present pictures of an "in vivo" animal study illustrating the invention. The model of the experiment was designed to represent a chronic dessicated eschar (a worst case senario for an enzymatic-chemical debridement). The testing animals were young pigs of 20-25 kg of weight. Under general anesthesia and proper monitoring (according to GAP) the bristles along the spine were shaved. The different test areas were marked and photographed. A series of deep contact burns, 2 cm in diameter, were left to desiccate for three days, creating a dry eschar, very similar to a typical old, chronic wound eschar. A circular chamber 3 cm in diameter was glued around each area. The USS circular probe having a diameter of 1.2 cm that covers half of each eschar site was installed with its surface covering the lower part of the eschar at a distance of 3 millimeters. At this stage the chamber was filled with the test material (Debridase and saline) or with the control (saline). A third control site was a similar eschar treated with hydrated Debridase in an occlusive dressing without the use of USS.

Fig 5. represents the burn area inside the chamber with the USS probe in place covering the lower part of the eschar.
Fig 6. represents the burn area inside the chamber with the USS probe in place immersed in the dissolved Debridase after 20 minutes of USS activity (Debridase solution stained with blood).

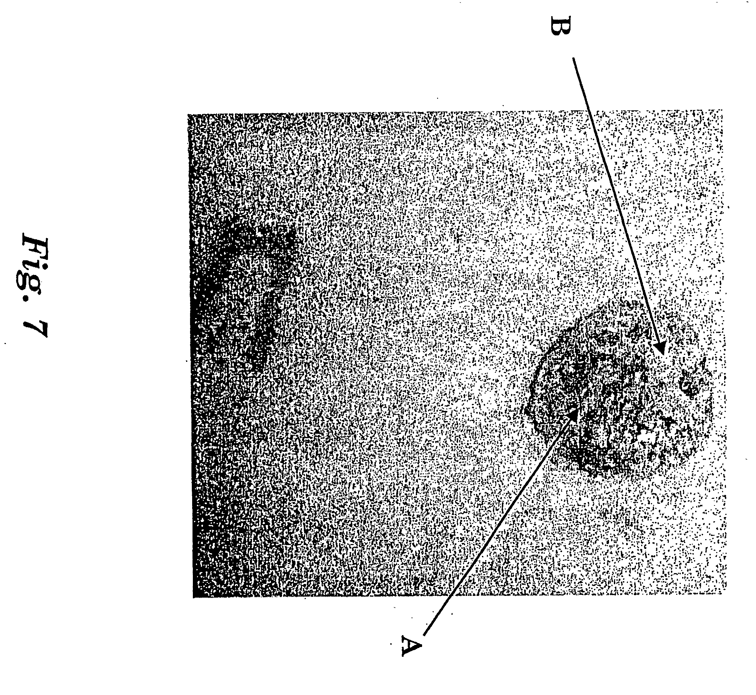

Fig 7- represents the burn area after 30 minutes of treatment with Debridase solution and USS. A completely debrided lower area A can be seen under the USS probe, contracted by the upper pole B, which the probe did not cover and where the eschar remained.

Fig 8. represents the burn area after 60 minutes of treatment with saline and USS. As can be seen, the eschar is completely intact without any signs of debridement.

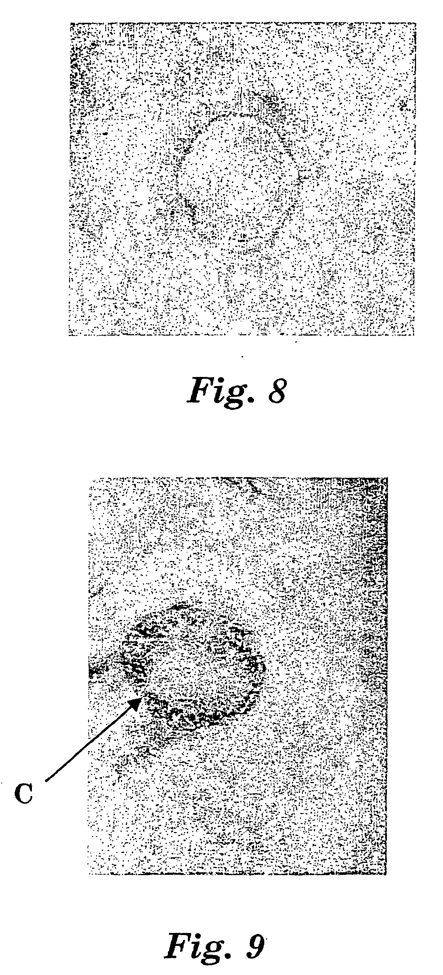

Fig. 9. represents the burn area after 60 minutes of treatment with hydrated Debridase in an occlusive dressing. Some initial debridement can be seen at the edges (marked by arrow C) where the eschar is thinner, but a thick reaming of the eschar remains in the center.

This animal study showed that the use of a debriding agent (represented by Debridase) alone produced a limited debridement as expected for the specific agent. The use of USS without any debriding agent did not have any debriding effect. However, the combination of an enzymatic debriding agent and a locally applied Ultra Sound produced clear and fast results (in a short period of 30 minutes).

While embodiments of the invention have been described by way of illustration, it will be apparent that many modifications, variations and adaptations can be made therein, without departing from the scope of the claims.

## Claims

1. A device (10; 30) for debridement of an area of skin to be debrided, comprising:
(a) an ultrasound probe (18; 28; 38; 42) for producing ultrasonic radiation; and
(b) a liquid retaining means (12; 24; 37; 41) coupled to said ultrasound probe (18; 28; 38; 42), said retaining means (12; 24; 37; 41) being adapted to be placed over the area of skin to be debrided, said retaining means (12; 24; 37; 41) containing a debriding agent and a solvent-acoustic medium, wherein said retaining means (12; 24; 37; 41) comprises an opening for enabling said debriding agent to contact the area of skin to be debrided and a seal (13; 23; 33; 46) for peripherally sealing said retaining means (12; 24; 37; 41) against the area of skin to be debrided;
wherein debridement of the area of skin to be debrided is performed through a combination of an effect of said ultrasonic radiation and an effect of the debriding agent.

2. The device (10; 30) of claim 1, wherein said liquid retaining means (12; 24; 37; 41) further comprises an inlet (20; 39; 44).

3. The device (10; 30) of claim 2, wherein said inlet (20, 39; 44) is for inserting at least one of said debriding agent, said acoustic medium, or a combination thereof.

4. The device (10; 30) of any of claims 1-3, wherein said liquid retaining means (12; 24; 37; 41) further comprises an outlet (22; 32; 47).

5. The device (10; 30) of claim 4, wherein said outlet (22; 32; 47) is for removing fluids.

6. The device (10; 30) of claim 4, wherein said outlet (22; 32; 47) is for at least creating a vacuum for sealing said opening (20) against said area of skin.

7. The device (10; 30) of claim 1, wherein said ultrasound probe (18; 28; 38; 42) comprises a piezoelectric ultrasound source unit.

8. The device (10; 30) of claim 1, further comprising a spacing means (26; 31; 41) for maintaining said ultrasound probe (18; 28; 38; 42) at a distance from the area of skin to be debrided.

9. The device (10; 30) of claim 8, wherein said spacing means (26; 31; 41) is constituted by or is provided within said liquid retaining means (12; 24; 37; 41).

10. The device (10; 30) of claim 1, wherein said debriding agent and said acoustic medium form a liquid debriding agent in combination.

11. The device (10; 30) of claim 1, wherein said debriding agent includes at least one of a chemical debriding agent or an enzymatic debriding agent.

12. The device (10; 30) of claim 11, wherein said debriding agent comprises at least one Bromelain derivative.

13. The device (10; 30) of claim 12, wherein said at least one Bromelain derivative comprises Debridase.

## Patentansprüche

1. Vorrichtung (10; 30) zur Wundtoilette eines zu reinigenden Hautbereichs, umfassend:
(a) eine Ultraschallsonde (18; 28; 38; 42) zum Erzeugen von Ultraschallwellen; und
(b) ein mit der Ultraschallsonde (18; 28; 38; 42) verbundenes Flüssigkeitsbehältermittel (12; 24; 37; 41), wobei das Behältermittel (12; 24; 37; 41) dafür beschaffen ist, über dem zu reinigenden Hautbereich angeordnet zu werden, wobei das Behältermittel (12; 24; 37; 41) ein Reinigungsagens und ein Lösemittel-Akustikmedium enthält,
wobei das Behältermittel (12; 24; 37; 41) eine Öffnung, die es dem Reinigungsagens ermöglicht, den zu reinigenden Hautbereich zu kontaktieren, und eine Dichtung (13; 23; 33; 46), mit dem das Behältermittel (12; 24; 37; 41) entlang des Umfangs gegen den zu reinigenden Hautbereich abgedichtet wird, umfasst;
wobei die Wundtoilette des zu reinigenden Hautbereichs durch eine Kombination eines Effekts der Ultraschallwellen und eines Effekts des Reinigungsagens' vorgenommen wird.

2. Vorrichtung (10; 30) nach Anspruch 1, wobei das Flüssigkeitsbehältermittel (12; 24; 37; 41) des Weiteren einen Einlass (20; 39; 44) umfasst.

3. Vorrichtung (10; 30) nach Anspruch 2, wobei der Einlass (20; 39; 44) zum Einfüllen wenigstens des Reinigungsagens' oder des Akustikmediums oder einer Kombination aus beiden dient.

4. Vorrichtung (10; 30) nach einem der Ansprüche 1-3, wobei das Flüssigkeitsbehältermittel (12; 24; 37; 41) des weiteren einen Auslass (22; 32; 47) umfasst.

5. Vorrichtung (10; 30) nach Anspruch 4, wobei der Auslass (22; 32; 47) zum Ablassen von Fluida dient.

6. Vorrichtung (10; 30) nach Anspruch 4, wobei der Auslass (22; 32; 47) dazu dient, wenigstens ein Vakuum zum Abdichten der Öffnung (20) gegen den Hautbereich zu erzeugen.

7. Vorrichtung (10; 30) nach Anspruch 1, wobei die Ultraschallsonde (18; 28; 38; 42) eine piezoelektrische Ultraschallquelleneinheit umfasst.

8. Vorrichtung (10; 30) nach Anspruch 1, die des Weiteren ein Abstandshaltermittel (26; 31; 41) umfasst, das dazu dient, die Ultraschallsonde (18; 28; 38; 42) in einem Abstand zu dem zu reinigenden Hautbereich zu halten.

9. Vorrichtung (10; 30) nach Anspruch 8, wobei das Abstandshalteimittel (26; 31; 41) durch das Flüssigkeitsbehältermittel (12; 24; 37; 41) gebildet wird oder innerhalb des Flüssigkeitsbehältermittels (12; 24; 37; 41) angeordnet ist.

10. Vorrichtung (10; 30) nach Anspruch 1, wobei das Reinigungsagens und das Akustikmedium zusammen ein flüssiges Reinigungsagens bilden.

11. Vorrichtung (10; 30) nach Anspruch 1, wobei das Reinigungsagens ein chemisches Reinigungsagens und/oder ein enzymatisches Reinigungsagens enthält.

12. Vorrichtung (10; 30) nach Anspruch 11, wobei das Reinigungsagens wenigstens ein Bromelainderivat umfasst.

13. Vorrichtung (10; 30) nach Anspruch 12, wobei das wenigstens eine Bromelainderivat Debridase umfasst.

## Revendications

1. Dispositif (10 ; 30) de débridement d'une zone de peau à débrider, comprenant :
(a) une sonde à ultrasons (18 ; 28 ; 38 ; 42) pour produire une radiation ultrasonique ; et
(b) un moyen de rétention de liquide (12 ; 24 ; 37 ; 41) couple à ladite sonde à ultrasons (18 ; 28 ; 38 ; 42), ledit moyen de rétention (12 ; 24 ; 37 ; 41) étant apte à être placé sur la zone de peau à débrider, ledit moyen de rétention (12 ; 24 ; 37 ; 41) contenant un agent de débridement et un milieu acoustique-solvant, dans lequel ledit moyen de rétention (12 ; 24 ; 37 ; 41) comprend une ouverture permettant au dit agent de débridement de se mettre en contact avec la zone de peau à débrider et un joint (13 ; 23 ; 33 ; 46) servant à sceller sur sa périphérie ledit moyen de rétention (12 ; 24 ; 37 ; 41) contre la zone de peau à débrider ;
dans lequel le débridement de la zone de peau à débrider est réalisé par combinaison d'un effet de ladite radiation ultrasonique et d'un effet de l'agent de débridement.

2. Dispositif (10 ; 30) selon la revendication 1, dans lequel ledit moyen de rétention de liquide (12 ; 24 ; 37 ; 41) comprend en outre une entrée (20 ; 39 ; 44).

3. Dispositif (10 ; 30) selon la revendication 1, dans lequel ladite entrée (20 ; 39 ; 44) est destinée à insérer au moins un élément parmi ledit agent de débridement, ledit milieu acoustique ou une combinaison des deux.

4. Dispositif (10 ; 30) selon l'une quelconque des revendications 1 à 3, dans lequel ledit moyen de rétention de liquide (12 ; 24 ; 37 ; 41) comprend en outre une sortie (22 ; 32 ; 47).

5. Dispositif (10 ; 30) selon la revendication 4, dans lequel ladite sortie (22 ; 32 ; 47) est destinée à éliminer les fluides.

6. Dispositif (10 ; 30) selon la revendication 4, dans lequel ladite sortie (22 ; 32 ; 47) est destinée au moins à créer un vide pour sceller ladite ouverture (20) contre ladite zone de peau.

7. Dispositif (10 ; 30) selon la revendication 1, dans lequel ladite sonde à ultrasons (18 ; 28 ; 38 ; 42) comprend une unité piézoélectrique de source d'ultrasons.

8. Dispositif (10 ; 30) selon la revendication 1, comprenant en outre un moyen d'espacement (26 ; 31 ; 41) servant à maintenir ladite sonde à ultrasons (18 ; 28 ; 38 ; 42) à distance de la zone de peau à débrider.

9. Dispositif (10 ; 30) selon la revendication 8, dans lequel ledit moyen d'espacement (26 ; 31 ; 41) est constitue par ou est installé dans ledit moyen de rétention de liquide (12 ; 24 ; 37 ; 41).

10. Dispositif (10 ; 30) selon la revendication 1, dans lequel ledit agent de débridement et ledit milieu acoustique forment en combinaison un agent de débridement liquide.

11. Dispositif (10 ; 30) selon la revendication 1, dans lequel ledit agent de débridement comprend au moins un agent de débridement chimique ou un agent de débridement enzymatique.

12. Dispositif (10 ; 30) selon la revendication 11, dans lequel ledit agent de débridement comprend au moins un dérivé de broméline.

13. Dispositif (10 ; 30) selon la revendication 12, dans lequel ledit au moins un dérivé de broméline contient du Débridase.
